# EUROPEAN PATENT APPLICATION

(11) **EP 4 806 379 A1**
(43) Date of publication of application: **16.09.2026**
(21) Application number: 24889044.4
(22) Date of filing: 01.11.2024
(51) Int. Cl.: A61B 34/10, A61B 34/30

(54) **METHOD AND APPARATUS FOR PLANNING CUTTING PATH FOR SURGICAL ROBOT**

(30) Priority: 07.11.2023 KR 20230153066
(71) Applicant: Curexo, Inc., Seoul 05814 (KR)
(72) Inventor: KIM, Bong Oh, Seoul 05814 (KR); PARK, Cheon Man, Seoul 05814 (KR)
(74) Representative: Stöckeler, Ferdinand
(86) International application number: PCT/KR2024/017047
(87) International publication number: WO 2025/100856

(57) **Abstract**

Disclosed are a method and apparatus for planning a cutting path of a surgical robot, the method including identifying implant planning information including a placement position and a placement pose of an implant planned with respect to a virtual bone model corresponding to a target bone; generating a cut section of the bone model based on the implant planning information; selecting a standard cutting path corresponding to a cut section of the bone model among one or more standard cutting paths pre-stored in the computing device; and generating a modified cutting path by modifying the selected standard cutting path based on an outer line of the cut section.

Thus, a patient-customized cutting path can be quickly planned based on the pre-stored standard cutting path.

## Description

### [TECHNICAL FIELD]

The disclosure relates to a technology for planning a cutting path of a surgical robot, and more particularly, to a method for planning a cutting path for a bone to be cut by a surgical robot to place an implant during artificial joint replacement using the surgical robot, and an apparatus for performing the same.

### [BACKGROUND ART]

Robotic surgery may be largely classified into passive robotic surgery, semi-active robotic surgery, and active robotic surgery. Passive robotic surgery refers to a method in which a surgeon operates a robot in person throughout the surgical process. Semi-active robotic surgery refers to a method in which a surgeon performs processes such as cutting using a robot, but a robot limits the movement of a surgical tool by the surgeon along a predetermined path through haptic feedback or the like during the processes. Meanwhile, active robotic surgery refers to a method in which a robot automatically performs surgery according to a planned cutting path without intervention of a surgeon.

As described above, since a robot actively cuts a bone during active robotic surgery, it is important to plan a cutting path suitable for the implant to be placed.

In the related art, a library in which cutting paths are defined according to the shapes or sizes of the implants is prepared in advance, and when a planner determines the shape and size of an implant to be placed on a target bone, a cutting path corresponding to the shape and size of the implant is selected from the library and used. Such a library-based cutting path has limitations in providing patient-customized surgery because it fails to consider the diversity of bone shapes according to patients.

Further, the cutting path is typically generated for an area is slightly smaller than the implant shape with a predetermined cutting margin to prevent damage to soft tissue, which causes a residual bone area to remain considerably uncut. Such a residual bone area needs to be additionally cut and finally finished by a surgeon in person, thereby not only causing inconvenience but also prolonging surgery. The cutting margin for minimizing the residual bone area varies depending on the specific position of the implant. However, it is difficult to set a conventional library-based cutting path considering all possible cases of minimum cutting margins corresponding to various implant positions, and thus there is a problem that the residual bone area is substantially large.

### [DISCLOSURE]

### [TECHNICAL PROBLEM]

The disclosure has been conceived to solve the problems of the related art as described above, and an aspect of the disclosure is to provide a method and apparatus for planning a cutting path of a surgical robot, which can generate a patient-customized cutting path in consideration of diversity of bone shapes according to patients and minimize a residual bone area.

### [TECHNICAL SOLUTION]

According to an embodiment of the disclosure, a method for planning a cutting path of a surgical robot, which is performed by a computing device, includes: identifying implant planning information including a placement position and a placement pose of an implant planned with respect to a virtual bone model corresponding to a target bone; generating a cut section of the bone model based on the implant planning information; selecting a standard cutting path corresponding to a cut section of the bone model among one or more standard cutting paths pre-stored in the computing device; and generating a modified cutting path by modifying the selected standard cutting path based on an outer line of the cut section.

Here, the generating the modified cutting path may include expanding the standard cutting path toward the outer line of the cut section.

Meanwhile, the standard cutting path may include a plurality of unit cutting paths, and may include a plurality of path points corresponding to start points and end points of the unit cutting paths.

In this case, the generating the modified cutting path may include generating a first modified cutting path by changing a length of one or more unit cutting paths.

Here, the generating the first modified cutting path may include: recognizing an intersection point between an extension line of the unit cutting path and the outer line of the cut section; determining a changed position of the path point based on the intersection point; and modifying the unit cutting path by applying the path point whose position is changed to the changed position.

Meanwhile, the method may further include identifying a movement limit distance over which the cutting tool can continuously move at once in a predetermined direction based on pre-stored information regarding the cutting tool of the surgical robot, wherein the determining the changed position of the path point may include determining the changed position in consideration of a movement direction of the cutting tool on the unit cutting path and the movement limit distance of the cutting tool.

In this case, in consideration of a cutting margin region preset for protecting surrounding tissues of the target bone, the changed position of the path point is determined such that the changed position is not located in a preset cutting margin region.

Further, the generating the modified cutting path further may include: determining whether to additionally modify the first modified cutting path by comparing an area of the cut section with an area of a cutting region predicted according to the first modified cutting path; and generating a second modified cutting path by increasing the number of the unit cutting paths constituting the first modified cutting path according to a result of the determination.

Here, the method may further include identifying a movement limit distance over which the cutting tool of the surgical robot can continuously move at once in a predetermined direction based on pre-stored information regarding the cutting tool of the surgical robot, wherein the generating of the second modified cutting path may include: determining an additional position at which a new path point is to be added considering the outer line of the cut section and the movement limit distance of the cutting tool; generating a new path point at the additional position; and adding a new unit cutting path to the first modified cutting path using the new path point.

In this case, the determining the additional position at which the new path point is to be added may include preventing the path point from being added within a range less than or equal to a predetermined distance from the outer line of the cut section.

In addition, the determining the additional position at which the new path point is to be added may include determining the additional position as a position where a movement amount of the cutting tool corresponding to the predetermined direction from an existing path point does not exceed the movement limit distance.

According to another embodiment of the disclosure, an apparatus for planning a cutting path of a surgical robot includes: a memory unit configured to store one or more standard cutting paths and implant planning information including a placement position and a placement pose of an implant planned with respect to a virtual bone model corresponding to a target bone; and a processor, the processor being configured to: generate a cut section of the bone model based on the implant planning information; select a standard cutting path corresponding to a cut section of the bone model among the one or more stored standard cutting paths; and generate a modified cutting path by modifying the selected standard cutting path based on an outer line of the cut section.

### [ADVANTAGEOUS EFFECTS]

As described above, according to the disclosure, a surgical effect can be further improved by automatically generating a patient-customized cutting path in consideration of diversity of bone shapes according to patients.

In addition, according to the disclosure, a patient-customized cutting path can be quickly planned by expanding a cutting path based on a pre-stored standard cutting path.

Furthermore, according to the disclosure, a cutting path is generated to minimize a residual bone area remaining after cutting by a surgical robot, thereby simplifying a surgical process and effectively preventing surgical delay.

### [DESCRIPTION OF DRAWINGS]

FIG. 1 is a diagram illustrating a schematic configuration of an arthroplasty robotic surgery system including an apparatus for planning a cutting path of a surgical robot according to an embodiment of the disclosure;
FIG. 2 is a block diagram illustrating a detailed configuration of an apparatus for planning a cutting path according to an embodiment of the disclosure;
FIG. 3 is a flowchart illustrating a method for planning a cutting path of a surgical robot according to an embodiment of the disclosure;
FIGS. 4A and 4B are diagrams illustrating a standard cutting path according to an embodiment of the disclosure;
FIG. 5 is a diagram for explaining a concept regarding a movement limit distance of a cutting tool;
FIG. 6 is a flowchart illustrating a detailed process of generating a first modified cutting path of FIG. 3;
FIGS. 7 and 8 are diagrams illustrating examples of modifying the standard cutting path without adding path points according to an embodiment of the disclosure, respectively;
FIG. 9 is a diagram for explaining an example of determining a change position of a path point when modifying a zigzag-shaped standard cutting path as in FIG. 8;
FIG. 10 is a flowchart illustrating a detailed process of generating a second modified cutting path of FIG. 3;
FIGS. 11 and 12 are diagrams illustrating examples of modifying the standard cutting path by adding new path points according to an embodiment of the disclosure, respectively; and
FIGS. 13A and 13B are diagrams for explaining a method for adding longitudinal path points according to an embodiment of the disclosure.

### [BEST MODE]

Hereinafter, specific embodiments of the disclosure will be described with reference to the drawings. However, in the following description and the accompanying drawings, detailed descriptions of well-known functions or configurations that may obscure the gist of the disclosure will be omitted. In addition, it should be noted that the same components are represented by the same reference numerals as much as possible throughout the drawings.

An apparatus for planning a cutting path of a surgical robot according to the disclosure plans a cutting path for a target bone to be cut by a surgical robot to place an implant during artificial joint replacement. The artificial joint replacement set forth herein includes total knee replacement, partial knee replacement, and hip joint replacement. Hereinafter, total knee replacement among the artificial joint replacements will be described by way of example.

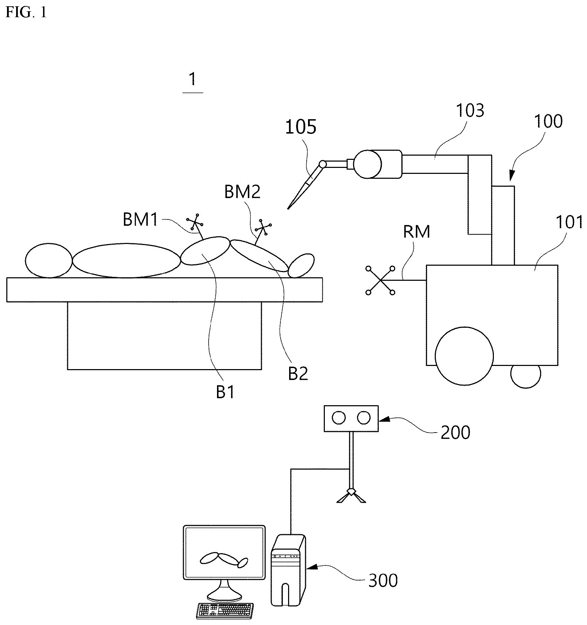

FIG. 1 is a diagram illustrating a schematic configuration of an arthroplasty robotic surgery system 1 including an apparatus for planning a cutting path of a surgical robot according to an embodiment of the disclosure.

Referring to FIG. 1, the arthroplasty robotic surgery system 1 includes bone markers BM1 and BM2 fixed to target bones B1 and B2, a surgical robot 100, a tracking device 200, and an apparatus 300 for planning a cutting path of a surgical robot (hereinafter referred to as a cutting path planning apparatus).

The target bones B1 and B2 refer to bones that are targets of robotic surgery, and FIG. 1 shows a femur B1 and a tibia B2 as an example. On the femur B1 and the tibia B2, bone markers BM1 and BM2, which serve as references for tracking the positions of the femur B1 and the tibia B2 during surgery, are fixedly mounted, respectively.

The surgical robot 100 is a robot that performs surgery for joint replacement and is composed of a robot base 101 and a robot arm 103. Various cutting tools 105 for performing bone cutting and the like including a bur may be coupled to an end effector at an end of the robot arm 103. As the cutting tool 105, various types of tools such as a milling cutter that performs cutting through rotational motion and a sawblade cutter that performs cutting through translational motion may be applied.

In addition, the surgical robot 100 may be equipped with various sensors capable of sensing state information of the surgical robot 100 such as a load applied to the cutting tool and an operating speed of the robot. On the base 101 of the surgical robot 100, a robot marker RM serving as a reference for tracking the position of the surgical robot 100 during surgery is fixed.

For reference, passive or active type optical markers may be applied as the bone markers BM1 and BM2 and the robot marker RM. The optical marker includes a plurality of bar members in the form of branches that branch in different directions with respect to a center point, and a ball marker may be formed at ends of each bar. This is only an example of the shape of the optical marker, and the optical marker may be implemented in various other known shapes.

The tracking device 200 is for tracking the positions and poses of the bone markers BM1 and BM2 fixed to the target bones B1 and B2 and the robot marker RM fixed to the surgical robot 100, and may be implemented as an optical tracking system (OTS). For reference, the optical tracking system refers to equipment capable of tracking markers using two infrared cameras and determining positions and poses in a three-dimensional space in real time by calculating distances based on triangulation. Since the tracking principle of such an optical tracking system is widely known, a detailed description thereof will be omitted.

The cutting path planning apparatus 300 plans a cutting path of the surgical robot 100 for target bones B1 and B2 requiring cutting in order to place an implant during joint replacement. Planning of the cutting path may be performed before robotic surgery or may be performed by a plan change during surgery. The cutting path includes position information indicating positions to which the surgical robot 100 is to move in a process in which the surgical robot 100 performs cutting while moving along the cutting path from a start point to an end point, and pose information of the surgical robot 100 at the corresponding position. The cutting path may be generated including relative position information and pose information of the surgical robot 100 based on an implant coordinate system. For reference, the cutting path includes not only a path through which a bone is directly cut by the cutting tool 105 of the surgical robot 100 but also a movement path for the surgical robot 100 to approach the bone for cutting.

The cutting path planning apparatus 300 may be implemented as a computing device including a processor for planning a cutting path, a display for displaying the planned cutting path, and a memory for storing generated cutting path information. Although FIG. 1 illustrates the cutting path planning apparatus 300 as being physically separated from the surgical robot 100 and implemented as a separate device, in some cases, the processor and the memory of the cutting path planning apparatus 300 may be provided in the surgical robot 100, the display may be connected to and installed together with the tracking device 200, and various information may be transmitted and received through a communication module. In addition, as described above, since the planning of the cutting path can be performed even before surgery, the cutting path planning apparatus 300 may not necessarily be located in the same space as the surgical robot 100 and the tracking device 200 required during surgery, but may be located in a remote space. In this case, the cutting path data generated by the cutting path planning apparatus 300 may be transmitted to the surgical robot 100 using various wired/wireless communication methods so that the surgical robot 100 can perform surgery according to the planned cutting path.

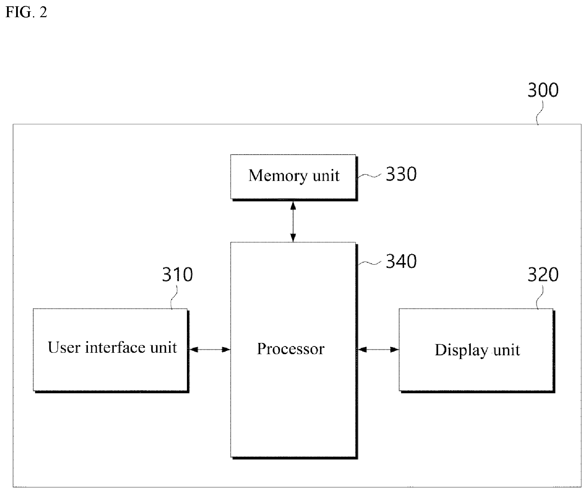

FIG. 2 is a block diagram illustrating a detailed configuration of a cutting path planning apparatus 300 according to an embodiment of the disclosure. Referring to FIG. 2, the cutting path planning apparatus 300 according to an embodiment of the disclosure includes a user interface unit 310, a display unit 320, a memory unit 330, and a processor 340.

The user interface unit 310 refers to a module for receiving various inputs from a user in the process of planning a cutting path of the surgical robot 100, and may be implemented using various input devices such as a mouse, a keyboard, a keypad, a button, and a pendant.

The display unit 320 is for displaying various information including images, graphics, and text on a screen, and may be implemented as a liquid crystal display (LCD) panel, a light emitting diode (LED) panel, an organic light emitting diode (OLED) panel, or the like. In addition, the user interface unit 310 and the display unit 320 may be integrated and implemented as one device such as a touch screen. The display unit 320 displays various GUIs and planning outputs provided in the process of planning an implant position and pose and the process of planning a cutting path of the surgical robot 100, including an implant model, 2D images and 3D models of a target bone, and the cutting path of the surgical robot 100.

The memory unit 330 is implemented as a memory device such as RAM, flash memory, or EPROM, and may store at least one computer program code executed by the processor 340. Such computer program code may be loaded into the memory unit 330 from a floppy drive, a disk, a tape, a DVD/CD-ROM drive, a memory card, and the like separate from the memory unit 330. In addition, the memory unit 330 stores CT images or MRI images of a patient captured before surgery, an implant library storing implant attribute information such as implant type/shape/length/size, a standard cutting path pre-generated based on an implant volume, information about a cutting tool including cutting characteristics of a cutting tool such as a type of the cutting tool mounted on the robot arm of the surgical robot 100 and a cutting depth, a cutting width, and a movement limit distance according to a predetermined direction, and other various reference information applied in the process of planning a cutting path of the surgical robot 100.

The processor 340 executes program code stored in the apparatus to perform a cutting path planning procedure of the surgical robot 100 for a target bone based on a pre-generated and pre-stored standard cutting path. That is, the processor 340 plans the cutting path of the surgical robot 100 not by generating the cutting path from scratch, but by modifying the pre-stored standard cutting path corresponding to the target bone.

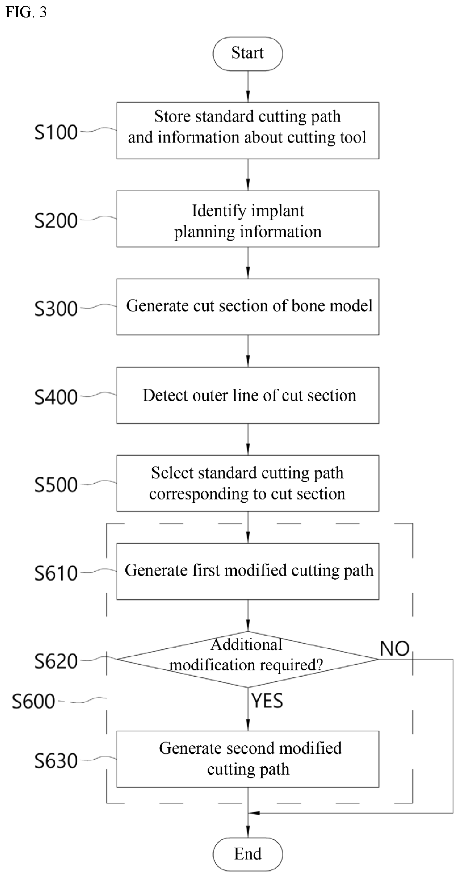

Hereinafter, a method for planning a cutting path of a surgical robot performed by the cutting path planning apparatus 300 will be described with reference to FIGS. 3 to 13.

FIG. 3 is a flowchart illustrating a method for planning a cutting path of a surgical robot according to an embodiment of the disclosure.

Referring to FIG. 3, it is assumed that standard cutting path data and information about a cutting tool are stored in the cutting path planning apparatus 300 (S100). The standard cutting path refers to a cutting path pre-generated based on an implant rather than the bone shape of a specific patient so as to be widely applicable to target bones of various patients, and is pre-generated considering characteristics of each implant such as the type, shape, and size of the implant, and cutting surfaces to be processed. The standard cutting path is generally generated to be located within the volume of the implant, except for a portion corresponding to an initial entry path for the surgical robot 100 to approach the bone for cutting among the entire path. This is to limit a cutting region that is substantially cut to be within the implant volume in order to protect soft tissues such as ligaments, blood vessels, muscles, and skin.

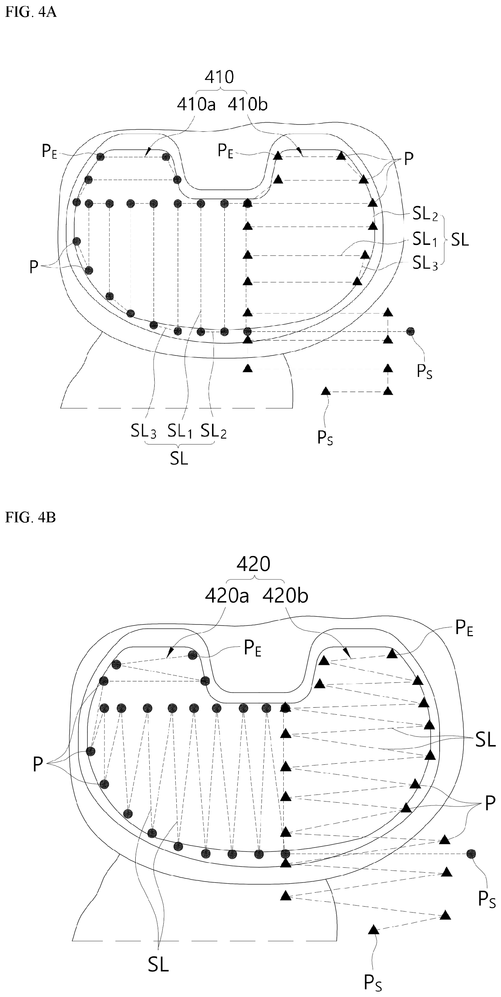

FIGS. 4A and 4B are diagrams illustrating a standard cutting path according to an embodiment of the disclosure;
Referring to FIGS. 4A and 4B, the standard cutting paths 410 and 420 are composed of a plurality of unit cutting paths SL and include a plurality of path points P corresponding to start points and end points of each unit cutting path SL.

In addition, FIGS. 4A and 4B respectively show examples of standard cutting paths 410 and 420 composed of two partial cutting paths for one cutting surface of a target bone. That is, FIG. 4A illustrates a standard cutting path 410 composed of a first partial cutting path 410a and a second partial cutting path 420a, and FIG. 4B illustrates a standard cutting path 420 composed of a first partial cutting path 410b and a second partial cutting path 420b. As such, the standard cutting path may be generated as one path for one cutting surface of the target bone, but may also include two or more partial cutting paths separated from each other for one cutting surface. In addition, the path point P is a point located on the cutting path, and except for the start point PS and the end point PE of the cutting path or each partial cutting path, each path point P is a connection point between two unit cutting paths SL and corresponds to a direction change point at which a direction of the cutting tool 105 changes.

FIG. 4A shows an example of a standard cutting path 410 composed of a combination of horizontal, longitudinal, and diagonal unit cutting paths SL perpendicular to one another. Specifically, it shows a standard cutting path composed of a combination of a first unit cutting path SL1 in which the cutting tool cuts while moving in the horizontal direction, a second unit cutting path SL2 in which the cutting tool cuts while moving in the longitudinal direction, and a third unit cutting path SL3 in which the cutting tool 105 cuts while moving in a diagonal direction including both a horizontal component and a longitudinal component. Meanwhile, FIG. 4B shows a zigzag-shaped standard cutting path composed of diagonal unit cutting paths SL including both a horizontal component and a longitudinal component. Here, it should be noted that directions such as horizontal, longitudinal, and diagonal directions do not mean the direction of the unit cutting path itself based on the implant coordinate system, but mean the direction in which the cutting tool moves along the corresponding unit cutting path, which is defined based on the cutting tool.

Although two types of standard cutting paths have been exemplified through FIGS. 4A and 4B, the standard cutting path is not limited thereto and may be provided in various forms according to a type of target bone such as a tibia or a femur, a position of a cutting surface on the target bone such as anterior region or a posterior region, and a type or size of an implant to be placed. For example, in FIG. 4B, a zigzag-shaped standard cutting path 420 composed only of a combination of diagonal unit cutting paths has been exemplified, but alternatively, a zigzag-shaped standard cutting path composed of two unit cutting paths parallel to each other and a unit cutting path connecting the two unit cutting paths in a diagonal direction is also possible. As such, the standard cutting path may be composed of various forms in which a first unit cutting path in which a cutting tool of a surgical robot moves in the horizontal direction on the corresponding path, a second unit cutting path in which the cutting tool moves in the longitudinal direction, or a third unit cutting path in which the cutting tool moves in a diagonal direction including both a horizontal component and a longitudinal component are combined.

One or more pre-generated standard cutting paths may be classified into categories based on positions of an implant and/or cutting surfaces and stored in the form of a library.

Meanwhile, information about a cutting tool may include cutting characteristics such as a type of the cutting tool, a diameter and size of the cutting tool, a cutting depth, and a cutting width, and in particular, may include a movement limit distance over which the cutting tool can continuously move in a specific direction in a single operation.

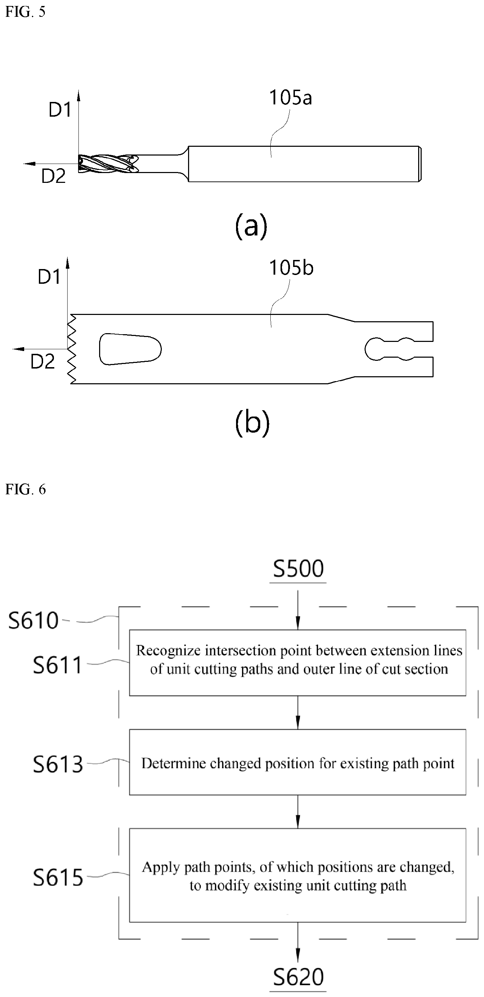

FIG. 5 is a diagram for explaining a concept regarding a movement limit distance of a cutting tool.

For a cutting tool, a movement limit distance, within which the tool can continuously move at once in a specific direction, that is, a movable distance per operation for a specific direction (or specific directional component) is determined according to the type of the tool. In this case, even for the same type of cutting tool, the specific value of the movement limit distance may vary depending on the diameter or size of the tool.

FIG. 5 illustrates the movement characteristics of the cutting tools 105, in which (a) shows a milling cutter 105a, and (b) shows a sawblade cutter 105b. First, referring to (a), when performing cutting, there is a limit to a distance that the milling cutter 105a can move continuously at once in a longitudinal direction (or longitudinal component) D2 defined with respect to the milling cutter 105a. Thus, even when the milling cutter 105a performs cutting while moving in a diagonal direction including both the horizontal component D1 and the longitudinal component D2, the milling cutter 105a is constrained by the movement limit distance in the longitudinal direction D2.

On the other hand, the sawblade cutter 105b has a limit to a distance that it can move continuously at once in the horizontal direction D1. Thus, even when the sawblade cutter 105b performs cutting while moving in a diagonal direction, the sawblade cutter 105 is constrained by the movement limit distance in the horizontal direction D1.

Due to the movement limit distance in a specific direction (or directional component) according to the type of the cutting tool, it is important to plan a cutting path such that the movement distance of the cutting tool in the specific direction, i.e., the movement distance of the cutting tool on a unit cutting path corresponding to the specific direction does not exceed the movement limit distance, which will be described later.

Subsequently, implant planning information regarding an implant to be placed on a target bone is identified (S200). The implant planning information may be generated based on a virtual 3D bone model corresponding to a target bone of a specific patient, and includes information about a placement position and a placement pose of the implant on the bone model. Here, the placement pose refers to an angle or a direction in which the implant is placed. The bone model for the target bone refers to a model reconstructed in 3D based on medical images of a patient obtained before surgery, for example, CT images, MRI images, and the like.

Determination of the placement position and pose of the implant may be made based on user input through the user interface unit 310. When a user selects an implant to be placed on the target bone from an implant library, the processor 340 generates a virtual model of the selected implant and displays the virtual model superimposed on the 3D bone model, and an appropriate placement position and pose such as a placement angle of the implant can be determined according to user input for moving the virtual implant model on the 3D bone model through the user interface unit 310.

Meanwhile, as described above, the determination of the placement position and pose of the implant may not be entirely dependent on user input but may be automatically made according to preset implant placement criteria. For example, it may be implemented such that the processor 340 identifies a position, shape, and size of a predetermined landmark in a 3D bone model and determines an implant type and an appropriate placement position and pose thereof based on the implant placement criteria information in response to identification results. The implant planning information determined by user input or the processor 340 may be stored and loaded.

As described above, when the implant planning information regarding the placement position and pose of the implant is identified, a cut section of the bone model is generated based on the implant planning information (S300). Here, the cut section refers to a section generated by virtually cutting a surface required to be cut for joint surgery in a bone model corresponding to a state before cutting. Although not actually cut, the processor 340 generates the cuts section in the bone model by predicting a post-cut state based on a 3D model of a target bone in a pre-cut state. The processor 340 may generate the cut section by predicting a state in which the corresponding cutting surface has been cut based on characteristics of an implant to be placed on a target bone such as a placement position and pose of the implant according to the implant planning information and the shape and size of the implant, and cutting characteristics of a cutting tool to perform cutting, for example, a depth of a single-cut depth and a cutting width.

When the generation of the cut section is completed, an outer line of the generated cut section is detected (S400). The outer line, as the outermost border of the cut section, may be detected based on various rule-based detection algorithms for recognizing line information based on a shape, color, and the like of an object shown in an image, or a deep learning-based algorithm trained to detect the outer line from the cut section through convolutional neutral network (CNN), R-CNN, YOLO, or the like.

Subsequently, the processor 340 selects a standard cutting path corresponding to the cut section of the bone model among one or more standard cutting paths pre-stored in the cutting path planning apparatus 300 (S500). Selection of the standard cutting path may be made considering the type and size of the implant, and the position and shape of the cut section based on the implant planning information.

For example, the selection criteria for selecting the standard cutting path may be preset according to the positions of respective cut sections and/or the type or size of an implant, and the processor 340 may select a standard cutting path according to the preset criteria or may select a standard cutting path input through the user interface unit 310. Alternatively, a standard cutting path frequently used in previous robotic surgeries may be selected based on surgical information history.

As such, when a standard cutting path is selected, a process of generating a modified cutting path by modifying the selected standard cutting path follows (S600). As described above, since the standard cutting path is typically generated to be located within the implant volume, modification of the standard cutting path is performed in a manner of expanding the standard cutting path toward an outer line of a cut section. Expansion of the standard cutting path is performed to widen the cutting area, and as will be described later, it may be performed by extending a length of a unit cutting path without adding path points constituting the standard cutting path, or by adding a new unit cutting path through the addition of a path point.

In this regard, the processor 340 first generates a first modified cutting path by changing lengths of one or more unit cutting paths constituting the standard cutting path in order to generate a modified cutting path based on the standard cutting path (S610). The first modified cutting path refers to a cutting path generated by expanding the standard cutting path by changing lengths of one or more unit cutting paths through changes in positions of existing path points without increasing the number of existing path points constituting the standard cutting path.

For reference, since modification of the standard cutting path is basically performed to expand the cutting area to reduce the residual bone area left after cutting by the surgical robot 100, modification may not be performed for a movement path in which cutting is not substantially performed among the standard cutting path, for example, a portion corresponding to an entry path for the surgical robot 100 to approach the target bone for cutting.

FIG. 6 is a flowchart illustrating a detailed process of step S610 of generating a first modified cutting path of FIG. 3, and FIGS. 7 and 8 are diagrams illustrating the process of generating the first modified cutting path according to an embodiment of the disclosure. Below, a process of generating the first modified cutting path based on the standard cutting path will be described with reference to FIGS. 6 to 8.

Referring to FIG. 6, the processor 340 recognizes intersection points between the extension lines of respective unit cutting paths constituting the standard cutting path and the outer line of the cut section (S611).

Subsequently, based on the intersection points, a position to be changed for one or more existing path points of the standard cutting path is determined (S613). In this case, the position to be changed is determined based on a movement direction of the cutting tool on a corresponding unit cutting path where each existing path point is located before change, and the movement limit distance information of the cutting tool.

The processor 340 applies the path points, of which the positions are changed to the change positions determined in step S613, to modify the existing unit cutting paths, thereby generating the first modified cutting path (S615).

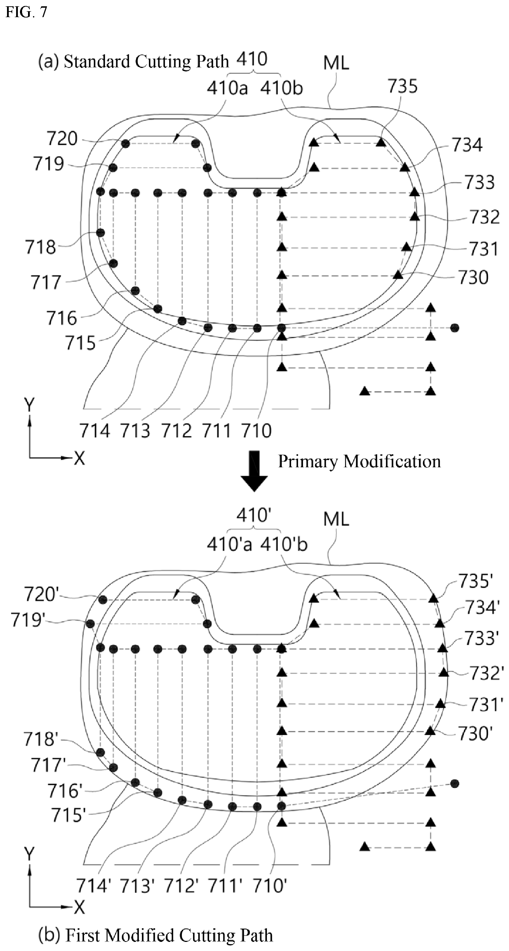

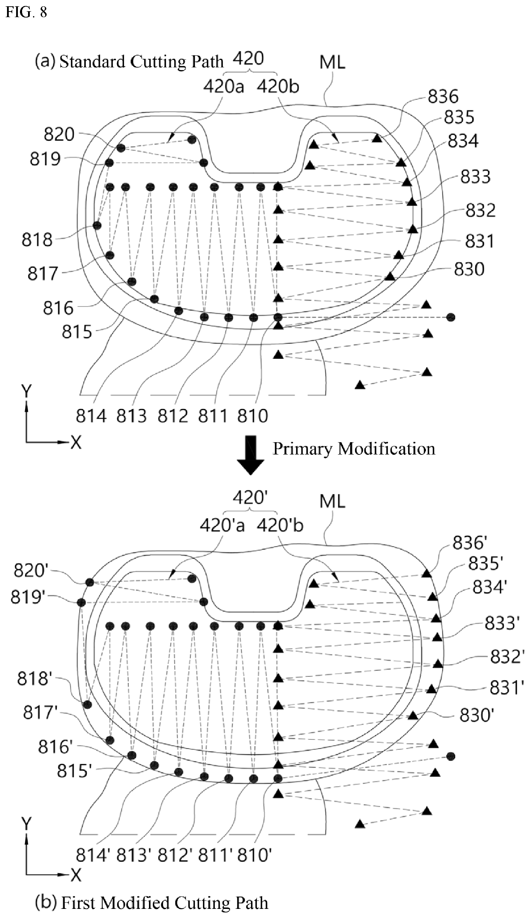

FIGS. 7 and 8 are diagrams illustrating examples of modifying the standard cutting path without adding path points, respectively. FIG. 7 shows an example of modifying the standard cutting path 410 composed mainly of the horizontal and longitudinal unit cutting paths shown in FIG. 4A, and FIG. 8 shows an example of modifying the zigzag-shaped standard cutting path 420 shown in FIG. 4B. For reference, in FIGS. 7 and 8, it is assumed that the movement of the cutting tool is constrained within a predetermined movement limit distance range with respect to the longitudinal movement.

First, referring to FIG. 7, positions of existing path points 710 to 720 and 730 to 735 are respectively changed to positions 710' to 720' and 730' to 735' corresponding to intersection points on an extension line of a unit cutting path SL and an outer line ML of a cut section. Accordingly, the lengths of the unit cutting paths SL are extended, and the standard cutting path is generally expanded toward the outer line ML of the cut section. In this case, the extension of the unit cutting path along which the cutting tool moves in the horizontal direction on the corresponding unit cutting path may be performed in a direction in which the cutting tool moves from an inner side to an outer side of the target bone, that is, toward the outer line ML of the cut section.

For reference, regarding the lower paths of the first partial cutting path 410a in FIG. 7, i.e., the paths including path points 710 to 718, an X-axis direction corresponds to a longitudinal movement of the cutting tool and a Y-axis direction corresponds to a horizontal movement of the cutting tool based on an implant coordinate system. Regarding the upper paths of the first partial cutting path 410a, i.e., the paths including path points 719 and 720, the X-axis direction corresponds to horizontal movement of the cutting tool and the Y-axis direction corresponds to the longitudinal movement of the cutting tool. On the other hand, regarding the second partial cutting path 410b, the X-axis direction corresponds to the horizontal movement of the cutting tool and the Y-axis direction corresponds to the longitudinal movement of the cutting tool.

In this case, regarding the unit cutting path SL corresponding to the horizontal movement, in which the movement of the cutting tool is not limited, among the corresponding unit cutting paths SL, the length thereof may be extended up to the outer line ML of the cut section. On the other hand, regarding the unit cutting path SL, in which the cutting tool moves in the longitudinal direction, among the corresponding unit cutting path SL, the extension is performed limitedly because this unit cutting path SL cannot exceed a preset longitudinal movement limit distance value of the cutting tool. Here, the longitudinal movement of the cutting tool includes diagonal movement including longitudinal component movement. Referring to FIG. 7, for example, when the movement of the cutting tool is performed in the longitudinal direction or includes a longitudinal movement component on the unit cutting path SL such as a unit cutting path SL connecting path points 711 and 712, a unit cutting path SL connecting path points 713 and 714, a unit cutting path SL connecting path points 715 and 716, or a unit cutting path SL connecting path points 717 and 718, the movement amount of the cutting tool is constrained by the movement limit distance. Thus, a change in position of the path point is limitedly performed so that the amount of movement in the longitudinal component can remain within the movement limit distance range.

Next, referring to FIG. 8, the standard cutting path 420 has a zigzag shape, which is different from that of FIG. 7, but the basic expansion concept is the same as that described with reference to FIG. 7.

Because each unit cutting path SL constituting the standard cutting path 420 of FIG. 8 is in a diagonal direction, each unit cutting path SL basically includes both horizontal movement components and longitudinal movement components of the cutting tool. Therefore, when extending the length of the diagonal unit cutting path SL, the position of the path point is changed so that the movement amount of the longitudinal component exists within a movement limit distance range of the cutting tool.

In other words, referring to FIG. 8, while satisfying a movement limit distance condition of the longitudinal component of the cutting tool, positions of existing path points 810 to 820 and 830 to 836 are respectively changed to positions 810' to 820' and 830' to 836' corresponding to intersection points between extension lines of the unit cutting path SL and the outer line ML of the cut section, and thus the length of the unit cutting path SL is expanded, thereby leading to the overall expansion of the standard cutting path toward the outer line ML of the cut section.

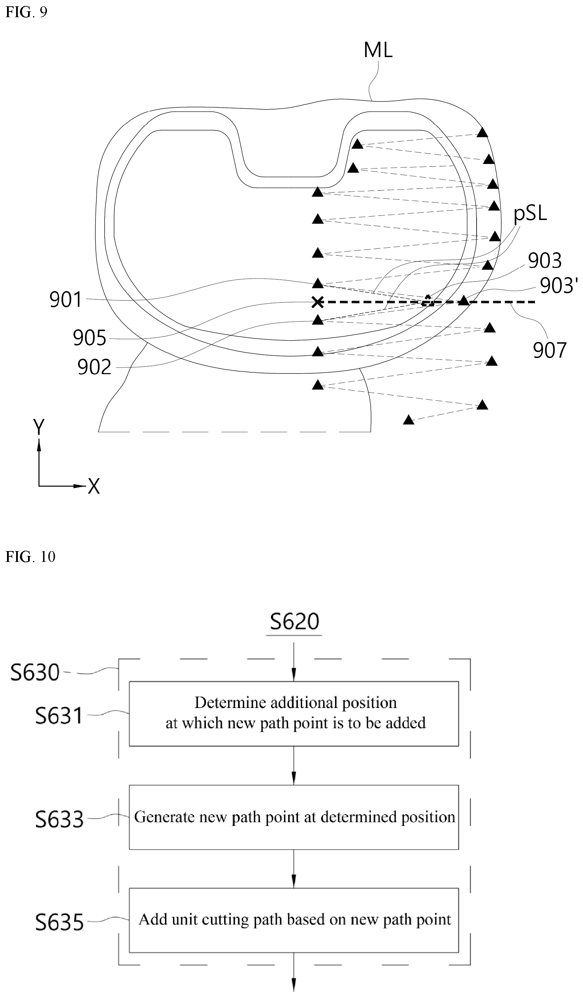

FIG. 9 is a diagram for explaining an example of determining a change position of a path point when modifying a zigzag-shaped standard cutting path as in FIG. 8. The path shown in FIG. 9 is a part of the cutting path, and in the partial cutting path, a Y-axis direction corresponds to longitudinal movement of the cutting tool based on the implant coordinate system. In addition, it will be assumed that the movement of the cutting tool is constrained within a predetermined limit distance range with respect to longitudinal movement.

Referring to FIG. 9, a virtual straight line 907 is generated between a midpoint 905 of two first and second path points 901 and 902 related to each other for the longitudinal movement of the cutting tool and an existing third path point 903 corresponding to an intersection of two existing unit cutting paths pSL. In addition, an intersection between the straight line 907 and the outer line ML of the cut section or a point positioned a predetermined distance inward from the intersection into the bone for soft tissue protection may be determined as a new changed position 903' of the existing path point 903. Through this, the standard cutting path may be expanded while satisfying the movement limit distance condition of the cutting tool for the longitudinal movement.

For reference, as described above, the intersection point between the extension line of each unit cutting path and the outer line of the cut section may be directly determined as the changed position of the path point. However, the changed position of the path point may also be determined as a position positioned a predetermined distance inward from the intersection point into the bone in consideration of a preset cutting margin region.

Here, the cutting margin region refers to a region set to be excluded from cutting for soft tissue protection, and may be set as a region positioned a predetermined distance inward from the outer line ML of the cut section into the target bone. A preset value may be applied for the size of the distance into the bone, and different values may be set according to the type of the cutting tool. Accordingly, the processor 340 expands the standard cutting path toward the outer line ML of the cut section, but may determine the changed position so that the changed position of the path point is not located in the preset cutting margin area.

As described above, when a primary modification process of modifying a standard cutting path by changing a length of a unit cutting path without increasing the number of path points is completed, the processor 340 compares an area of a cutting region predicted according to the first modified cutting path generated through the above process with an area of a cut section of a bone model to determine whether additional modification is required (S620). For reference, when predicting the cutting region according to the first modified cutting path, a path along which the cutting tool passes but no substantial cutting is performed, or a path along which the cutting tool redundantly passes to move to a next cutting position after cutting, is excluded from cumulative calculation in predicting the cutting region.

If the area of the cutting region predicted according to the first modified cutting path is smaller than the area of the cut section, or if a difference therebetween is larger than a preset value, the processor 340 may generate a second modified cutting path by modifying the first modified cutting path through secondary modification in which a new path point is added (S630). That is, the secondary modification is to modify the standard cutting path by increasing the number of unit cutting paths through addition of new path points.

FIG. 10 is a flowchart illustrating a detailed process of step S630 of generating a second modified cutting path of FIG. 3, FIGS. 11 to 13 are diagrams illustrating the process of generating the second modified cutting path according to an embodiment of the disclosure. Below, the process of generating the second modified cutting path will be described with reference to FIGS. 10 to 13.

First, referring to FIG. 10, the processor 340 determines an additional position at which a new path point is to be added considering the outer line of a cut section of a bone model and a movement limit distance according to a predetermined direction of a cutting tool (S631). In this case, the processor 340 may prevent generation of a new path point within a predetermined distance range from the outer line of the cut section in consideration of the aforementioned cutting margin region.

Subsequently, a new path point is generated at the determined position (S633) and a new unit cutting path is added to the first modified cutting path using the generated new path point (S635).

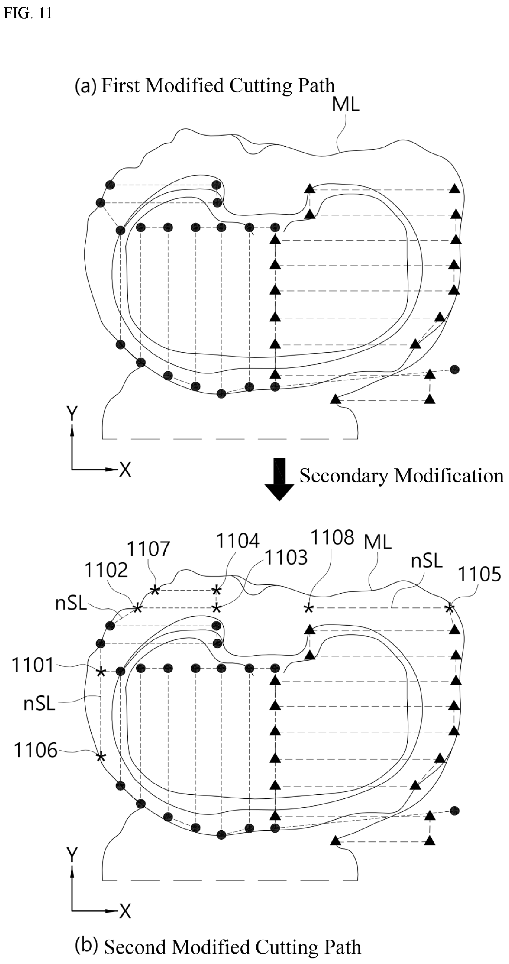

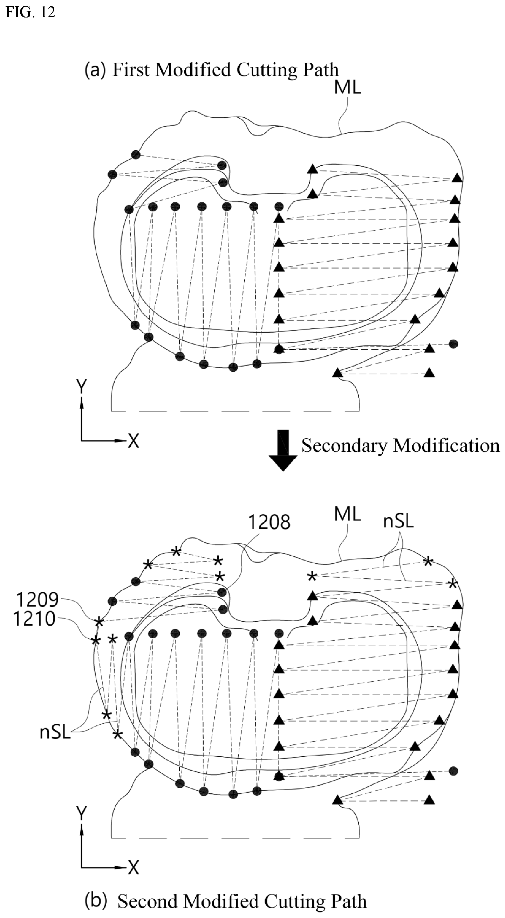

FIGS. 11 and 12 are diagrams illustrating the examples of modifying the standard cutting path by adding new path points, respectively. FIG. 11 shows an example of modification for a standard cutting path composed mainly of horizontal and longitudinal unit cutting paths as described in FIG. 7, and FIG. 12 shows an example of modification for a zigzag-shaped standard cutting path as described in FIG. 8. In FIGS. 11 and 12, it will be also assumed that the movement of the cutting tool is constrained within a predetermined limit distance range with respect to longitudinal movement. For reference, a newly added path point is illustrated as * to distinguish it from an existing path point.

In FIGS. 11 and 12, (a) shows a first modified cutting path in which primary modification of changing lengths of unit cutting paths with respect to a standard cutting path is completed, and (b) shows a state in which a second modified cutting path is generated by newly adding a unit cutting path based on a newly added path point.

Similar to changing the position of a path point, when a new path point is added, the new path point is added at a position where a movement distance, from an immediately preceding existing path point, in a predetermined direction (or directional component) of the cutting tool does not exceed a preset movement limit distance.

According to the embodiments of FIGS. 11 and 12, when adding a new path point, the processor 340 may first add a longitudinal path point to be located on a longitudinal movement line in which movement of the cutting tool is constrained by a movement limit distance, and subsequently add a horizontal path point to be located on a horizontal movement line of the cutting tool based on the added longitudinal path point. Meanwhile, the addition of the longitudinal path point and the horizontal path point is performed at positions that do not exceed the boundary of the outer line of the cut section.

In this regard, first, referring to FIG. 11, in the cutting path of (a), expansion of a cutting path through addition of a path point is performed because further expansion is impossible by only changing the length of the unit cutting path SL.

When adding new path points 1101 to 1108, it is important for new path points located on the longitudinal movement line where the movement of the cutting tool is contained limited that a movement amount of the cutting tool from an existing path point does not exceed a preset movement limit distance. As an example of generating new path points, the processor 340 first adds 1101, 1103, 1104, and 1108 corresponding to new path points on the longitudinal movement line such that a movement amount of the cutting tool from an immediately preceding existing path point does not exceed the preset movement limit distance. Subsequently, the processor 340 generates virtual lines starting from 1101, 1103, 1104, and 1108 toward the outer line ML of the cutting surface, respectively, and generates new path points 1102, 1105, 1106, and 1107 based on intersection points where the virtual lines meet the outer line ML of the cut section. New unit cutting paths nSL are generated by connecting the new path points 1101 to 1108.

For reference, the reference numerals of "1102" and "1105" are new path points included in a new unit cutting path nSL in which the cutting tool proceeds from an immediately preceding path point in a diagonal direction including both a horizontal movement component and a longitudinal movement component. These are constrained by a preset movement limit distance with respect to a longitudinal movement component of the cutting tool. However, since the longitudinal movement limit distance has already been taken into account when adding the path points 1103 and 1108, the path points 1102 and 1105 located on the same horizontal movement path of the cutting tool as the path points 1103 and 1108 also do not exceed the longitudinal movement limit distance of the cutting tool.

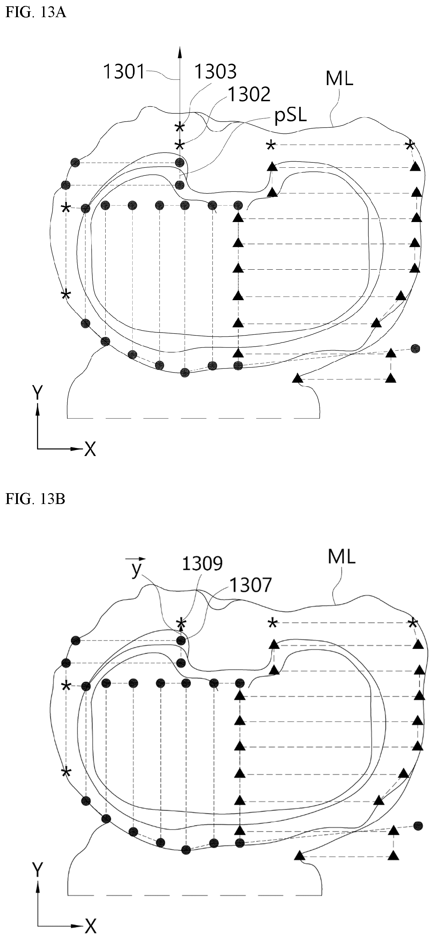

FIGS. 13A and 13B are diagrams illustrating a method of adding a longitudinal path point according to an embodiment of the disclosure.

First, referring to FIG. 13A, a virtual straight line 1301 extending an immediately preceding longitudinal unit cutting path pSL among existing unit cutting paths is generated, and one or more new longitudinal path points 1302 and 1303 located on a longitudinal movement line may be added at positions spaced apart on the straight line 1301 so as not to exceed the movement limit distance of a cutting tool.

Alternatively, as described above, a vector direction corresponding to a longitudinal direction on an implant coordinate system may be used without generating the virtual straight line 1301. That is, referring to FIG. 13B, a new longitudinal path point 1309 may be added at a position spaced apart from an immediately preceding path point 1307 on the longitudinal movement line among existing path points in a direction of a vector corresponding to the longitudinal direction in the implant coordinate system by a distance not exceeding the movement limit distance.

As described above, there may be several methods for adding a longitudinal path point, but all of these methods are the same in that a horizontal path point located on a horizontal movement line is generated based on an intersection point where a virtual horizontal movement line of the cutting tool starting from a newly added longitudinal path point meets an outer line of a cut section.

Meanwhile, adding a new path point in a zigzag-shaped cutting path may also be performed in the same manner as described with reference to FIG. 11.

In other words, according to the zigzag-shaped cutting path as in FIG. 12, since each unit cutting path has a diagonal direction including both a horizontal movement component and a longitudinal movement component of the cutting tool, it is necessary to generate a new path point such that the longitudinal movement component does not exceed the movement limit distance of the cutting tool.

Accordingly, when adding a new path point in the zigzag-shaped cutting path as in FIG. 12, the position of the new path point is determined so that a size of a longitudinal movement component from an immediately preceding path point to the new path point is limited to a preset movement limit distance of the cutting tool, and a size of a horizontal movement component is not particularly limited as long as it does not cross a boundary of the outer line of the cut section or a boundary of a preset cutting margin area (if applied).

Meanwhile, if the movement limit distance of the cutting tool is exceeded when generating a new unit cutting path by connecting an existing path point and an adjacent new path point, a new path point may be additionally generated in the middle. For example, if a new unit cutting path nSL is generated by connecting an existing path point 1208 and a new path point 1210 among the cutting paths of FIG. 12, and a movement distance corresponding to a longitudinal component of the cutting tool exceeds the movement limit distance of the cutting tool, the processor 340 may modify the cutting path to pass through a new path point 1209 added in the middle instead of generating the new unit cutting path nSL by connecting the path points 1208 and 1210.

Steps S300 to S600 of FIG. 3 described above indicate that they are performed for one cut section, and if a plurality of cut sections exist, steps S300 to S600 are repeatedly performed for each cut section.

The cutting path planning apparatus 300 may store a final cutting path for which modification is completed and display the final cutting path through the display unit 320, thereby allowing a user to verify the final cutting path.

As described above, according to the disclosure, by planning a cutting path customized for a cut section of a target bone based on modification of a standard cutting path, a surgical plan can be established more efficiently compared to planning a cutting path from scratch. In addition, a residual bone area remaining after cutting by a surgical robot can be minimized, thereby simplifying a surgical process and effectively preventing surgical delay.

When executed on a computer, the disclosure may also be implemented as various computer-readable recording media such as a magnetic storage medium, an optical reading medium, and a digital storage medium in which a computer program for performing the method for planning a cutting path of a surgical robot according to the disclosure is stored.

Although a few embodiments of the disclosure have been described, those skilled in the art will appreciate that various modifications and substitutions can be made to some embodiments without departing from the technical spirit of the disclosure. Therefore, the scope of protection of the disclosure should be construed as extending to the appended claims and equivalents thereof.

## Claims

1. A method for planning a cutting path of a surgical robot, which is performed by a computing device, the method comprising:
identifying implant planning information comprising a placement position and a placement pose of an implant planned with respect to a virtual bone model corresponding to a target bone;
generating a cut section of the bone model based on the implant planning information;
selecting a standard cutting path corresponding to a cut section of the bone model among one or more standard cutting paths pre-stored in the computing device; and
generating a modified cutting path by modifying the selected standard cutting path based on an outer line of the cut section.

2. The method of claim 1, wherein the generating the modified cutting path comprises expanding the standard cutting path toward the outer line of the cut section.

3. The method of claim 1, wherein the standard cutting path comprises a plurality of unit cutting paths, and comprises a plurality of path points corresponding to start points and end points of the unit cutting paths.

4. The method of claim 3, wherein the generating the modified cutting path comprises generating a first modified cutting path by changing a length of one or more unit cutting paths.

5. The method of claim 4, wherein the generating the first modified cutting path comprises:
recognizing an intersection point between an extension line of the unit cutting path and the outer line of the cut section;
determining a changed position of the path point based on the intersection point; and
modifying the unit cutting path by applying the path point whose position is changed to the changed position.

6. The method of claim 5, further comprising identifying a movement limit distance over which the cutting tool can continuously move at once in a predetermined direction based on pre-stored information regarding the cutting tool of the surgical robot,
wherein the determining the changed position of the path point comprises determining the changed position in consideration of a movement direction of the cutting tool on the unit cutting path and the movement limit distance of the cutting tool.

7. The method of claim 5, wherein, in consideration of a cutting margin region preset for protecting surrounding tissues of the target bone, the changed position of the path point is determined such that the changed position is not located in a preset cutting margin region.

8. The method of claim 4, wherein the generating the modified cutting path further comprises:
determining whether to additionally modify the first modified cutting path by comparing an area of the cut section with an area of a cutting region predicted according to the first modified cutting path; and
generating a second modified cutting path by increasing the number of the unit cutting paths constituting the first modified cutting path according to a result of the determination.

9. The method of claim 8, further comprising identifying a movement limit distance over which the cutting tool of the surgical robot can continuously move at once in a predetermined direction based on pre-stored information regarding the cutting tool of the surgical robot,
wherein the generating of the second modified cutting path comprises:
determining an additional position at which a new path point is to be added considering the outer line of the cut section and the movement limit distance of the cutting tool;
generating a new path point at the additional position; and
adding a new unit cutting path to the first modified cutting path using the new path point.

10. The method of claim 9, wherein the determining the additional position at which the new path point is to be added comprises preventing the path point from being added within a range less than or equal to a predetermined distance from the outer line of the cut section.

11. The method of claim 9, wherein the determining the additional position at which the new path point is to be added comprises determining the additional position as a position where a movement amount of the cutting tool corresponding to the predetermined direction from an existing path point does not exceed the movement limit distance.

12. An apparatus for planning a cutting path of a surgical robot, the apparatus comprising:
a memory unit configured to store one or more standard cutting paths and implant planning information comprising a placement position and a placement pose of an implant planned with respect to a virtual bone model corresponding to a target bone; and
a processor,
the processor being configured to:
generate a cut section of the bone model based on the implant planning information;
select a standard cutting path corresponding to a cut section of the bone model among the one or more stored standard cutting paths; and
generate a modified cutting path by modifying the selected standard cutting path based on an outer line of the cut section.
